(19) Europäisches Patentamt
European Patent Office
Office européen des brevets

(11) Veröffentlichungsnummer: **0 013 007**
**B1**

# (12) EUROPÄISCHE PATENTSCHRIFT

(45) Veröffentlichungstag der Patentschrift: **10.02.82**

(51) Int. Cl.³: **C 07 D 277/82**

(21) Anmeldenummer: **79105297.0**

(22) Anmeldetag: **20.12.79**

(54) **Verfahren zur Herstellung von 4-Methyl-2-aminobenzthiazol.**

(30) Priorität: **20.12.78 CH 12931/78**

(43) Veröffentlichungstag der Anmeldung: **09.07.80 Patentblatt 80/14**

(45) Bekanntmachung des Hinweises auf die Patenterteilung: **10.02.82 Patentblatt 82/6**

(84) Benannte Vertragsstaaten: **BE DE FR GB IT LU NL**

(56) Entgegenhaltungen:
**DE-A-2 601 700**
**FR-A-688 867**
**FR-A-727 410**
**FR-A-2 298 548**
**FR-A-2 357 553**
**US-A-1 911 085**
**US-A-1 984 885**

(73) Patentinhaber: **LONZA AG, Gampel/Wallis (CH)**

(72) Erfinder: **Squaratti, Armand, Neue Simplonstrasse 6, Brig (CH)**

(74) Vertreter: **Weinhold, Peter, Dr. Patentanwälte Dipl.-Ing. P. Wirth Dr. V. Schmied-Kowarzik et al, Dipl.-Ing. G. Dannenberg Dr. P. Weinhold, Dr. D. Gudel Dipl.-Ing. S. Schubert Siegfriedstr. 8, D-8000 München 40 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Verfahren zur Herstellung von 4-Methyl-2-aminobenzthiazol

Aus der FR-PS 23 57 553 ist bekannt, dass Aminobenthiazole aus Phenylthioharnstoffen mit Chlor in aprotischen Lösungsmitteln hergestellt werden können. Für den Ablauf der Reaktion ist ein Brom- oder Jodkatalysator erforderlich, und sein Weglassen führt zu wirtschaftlich uninteressanten Ausbeuten. Nach der US-PS 19 84 885 führt eine Umsetzung von o-Tolylthioharnstoff mit Chlor unter Verwendung von Nitrobenzol als Lösungsmittel zu Produkten, welche am Benzolring chloriert sind. Wird eine Ringschlussreaktion von Arylthioharnstoffverbindungen mittels Chlor durchgeführt, wie es in der US-PS 19 11 085 beschrieben ist, so wird eine Kernchlorierung nur dann vermieden, wenn im Ausgangsprodukt in Parastellung zum Thioharnstoffrest ein Substituent vorhanden ist. In der überwiegenden Zahl der Beispiele dieser US-Patentschrift wird das teurere Brom als Ringschlussreagenz verwendet. In der FR-PS 688 867 verwendet man für die Ringschlussreaktion von Arylthioharnstoffen in Lösungsmitteln, wie Chlorbenzol oder Tetrachlorkohlenstoff, z.B. Sulfurchlorid oder Chlor. Auch hier findet eine Kernchlorierung in denjenigen Fällen statt, wo kein Substituent in Parastellung zu der Thioharnstoffgruppe im Ausgangsprodukt vorhanden war.

Der Erfindung liegt die Aufgabe zugrunde, ein Verfahren zu schaffen, das die Herstellung von 4-Methyl-2-aminobenzthiazol auf einfache Art und Weise und in hoher Ausbeute ermöglicht. Die Ringschlussreaktion der entsprechenden o-Tolylthioharnstoffverbindung soll mit Hilfe von Chlor und ohne Katalysator möglich sein. Die Bildung von kernchlorierten Produkten soll unterdrückt und die Bildung von schwerlöslichen Zwischenprodukten, welche zur Verklumpung des Reaktionsgemisches führen, ausgeschaltet werden.

Die Lösung dieser Aufgabe erfolgt nach der Lehre des Patentanspruches.

Die erfindungsgemässe Reaktion folgt der Formel:

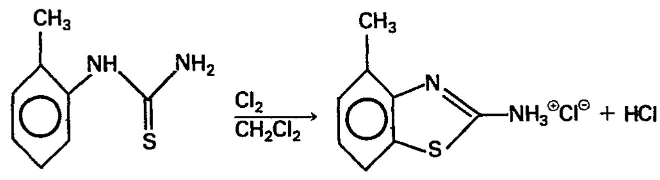


I II

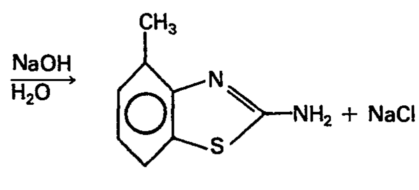


III

Das Verfahren wird so ausgeführt, dass man auf an sich bekannte Weise aus o-Toluidin und Ammonrhodanid bzw. Natriumrhodanid in wässriger Salzsäure den o-Tolythioharnstoff (I) herstellt. In einer erfindungsgemässen zweiten Stufe wird der o-Tolylthioharnstoff in der 3- bis 40-fachen Verdünnung in Methylenchlorid (Gew./Vol.) aufgeschlämmt und auf eine Temperatur zwischen −20 und +15 °C gekühlt. Unter Rühren wird Chlor in Mengen von 1 bis 2 Mol, bezogen auf o-Tolylthioharnstoff, in das Gemisch eingeleitet. Der sich bildende Chlorwasserstoff wird durch Kochen am Rückfluss während 1 bis 2 Stunden entfernt. Nach Beendigung der Chlorwasserstoffentwicklung kann das 4-Methyl-2-aminobenzthiazol-hydrochlorid (II) abfiltriert werden. Das Hydrochlorid wird in Wasser mit einem Alkali, vorzugsweise Natronlauge, zum 4-Methyl-2-aminobenzthiazol (III) umgesetzt und abfiltriert.

Das derart umgesetzte Produkt ist frei von Verunreinigungen und fällt als weisses, kristallines Pulver in hoher Ausbeute aus.

Das Verfahren gemäss der Erfindung gestattet die Herstellung von 4-Methyl-2-aminobenzthiazol ohne Anwendung eines zusätzlichen Katalysators, der seinerseits wieder aus dem Reaktionsgemisch entfernt und isoliert werden müsste. Eine Chlorierung am Benzolring erfolgt trotz des fehlenden Katalysators in keinem Fall.

Beispiel

73,5 g o-Tolylthioharnstoff, hergestellt nach bekannten Verfahren, wurde in 375 ml Methylenchlorid aufgeschlämmt und auf ca. 1 °C gekühlt. Durch ein Gaseinleitungsrohr wurden 32,5 g Chlorgas unter Rühren in das Reaktionsgemisch geleitet. Das 4-Methyl-2-aminobenzthiazol-hydrochlorid kristallisierte aus, wobei die Chlorwasserstoffentwicklung einsetzte. Nach Beendigung der Chlorwasserstoffentwicklung bei Rückflusstemperatur wurde das Reaktionsgemisch abgenutscht und getrocknet. Die Ausbeute betrug 85,0 g, entsprechend 95,8% 4-Methyl-2-aminobenzthiazol-hydrochlorid.

Durch Behandeln des Hydrochlorids mit wässriger Natronlauge konnten 67,3 g (96,8%) 4-Methyl-2-aminobenzthiazol gewonnen werden.

Die erfindungsgemäss hergestellten Produkte eignen sich in besonderer Weise zur Herstellung von Farbstoffen.

**Patentanspruch**

Verfahren zur Herstellung von 4-Methyl-2-aminobenzthiazol aus o-Tolylthioharnstoff mit Chlor als Ringschlussreagens, dadurch gekennzeichnet, dass die Reaktion ohne Katalysator in Methylenchlorid ausgeführt wird.

**Revendication**

Procédé de préparation de 4-méthyl-2-aminobenzothiazole à partir de o-tolyol-thio-urée avec de chlore comme agent de cydisation, caractérisé en ce que la réaction est effectuée sans catalyseur dans le chlorure de méthylène.

**Claim**

Process for preparing aminobenzothiazole from o-tolylthiourethane with chlorine as ring closure agent, characterized in that the reaction is carried out without any catalyst in methylene chloride.